# EUROPEAN PATENT SPECIFICATION

(11) **EP 1 251 138 B1**
(45) Date of publication and mention of the grant of the patent: **26.07.2006**
(21) Application number: 01109707.8
(22) Date of filing: 19.04.2001
(51) Int. Cl.: C07K 14/47, C12N 15/62, A61K 39/00

(54) **Prion protein dimers useful for vaccination**
Prion Proteindimere für Impfungen
Dimères de prions comme vaccin

(43) Date of publication of application: 23.10.2002
(73) Proprietor: Schätzl, Hermann, Dr., 82140 Olching (DE)
(72) Inventor: Schätzl, Hermann, Dr., 82140 Olching (DE)
(74) Representative: Schüssler, Andrea

(56) References cited:
- PRIOLA S.A. ET AL.: "A 60-kDa Prion Protein (PrP) with properties of both the normal and scrapie-associated forms of PrP*" J. BIOL. CHEM., vol. 270, no. 7, 17 February 1995 (1995-02-17), pages 3299-3305, XP002178941
- HARMEYER S ET AL.: "Synthetic peptide vaccines yield monoclonal antibodies to cellular and pathological prion proteins of ruminants." J. GENERAL VIROL., vol. 79, 1998, pages 937-945, XP002178942

### SEQUENCE LISTING

<110> Dr. Schätzl, Herrmann
<120> Prion protein dimers
<130> S1044
<140> EP 01109707.8
   <141> 2001-04-19
<160> 15
<170> PatentIn Ver. 2.1
<210> 1
   <211> 1320
   <212> DNA
   <213> Artificial Sequence
<220>
   <223> Description of Artificial Sequence:
   fusion protein
<220>
   <221> CDS
   <222> (1)..(1317)
<400> 1
   atg aga gga tcg cat cac cat cac cat cac gga tcc tgc aag aag cgg     48
   Met Arg Gly Ser His His His His His His Gly Ser Cys Lys Lys Arg
    1               5                  10                  15
   cca aag cct gga ggg tgg aac act ggc gga agc cga tac cct ggg cag     96
   Pro Lys Pro Gly Gly Trp Asn Thr Gly Gly Ser Arg Tyr Pro Gly Gln
               20                  25                  30
   ggg agc cct gga ggc aac cgt tac cca cct cag ggt ggc acc tgg ggg    144
   Gly Ser Pro Gly Gly Asn Arg Tyr Pro Pro Gln Gly Gly Thr Trp Gly
           35                  40                  45
   cag ccc cac ggt ggt ggc tgg gga caa ccc cat ggg ggc agc tgg gga    192
   Gln Pro His Gly Gly Gly Trp Gly Gln Pro His Gly Gly Ser Trp Gly
       50                  55                  60
   caa cct cat ggt ggt agt tgg ggt cag ccc cat ggc ggt gga tgg ggc    240
   Gln Pro His Gly Gly Ser Trp Gly Gln Pro His Gly Gly Gly Trp Gly
   65                  70                  75                  80
   caa gga ggg ggt acc cac aat cag tgg aac aag ccc agt aag cca aaa    288
   Gln Gly Gly Gly Thr His Asn Gln Trp Asn Lys Pro Ser Lys Pro Lys
                   85                  90                  95
   acc aac atg aag cac atg gcc ggc gct gct gcg gca ggg gcc gtg gtg    336
   Thr Asn Met Lys His Met Ala Gly Ala Ala Ala Ala Gly Ala Val Val
               100                 105                 110
   ggg ggc ctt ggt ggc tac atg ctg ggg agt gcc atg agc agg ccc atg    384
   Gly Gly Leu Gly Gly Tyr Met Leu Gly Ser Ala Met Ser Arg Pro Met
           115                 120                 125
   atc cat ttt ggc aac gac tgg gag gac cgc tac tac cgt gaa aac atg    432
   Ile His Phe Gly Asn Asp Trp Glu Asp Arg Tyr Tyr Arg Glu Asn Met
       130                 135                 140
   tac cgc tac cct aac caa gtg tac tac agg cca gtg gat cag tac agc     480
   Tyr Arg Tyr Pro Asn Gln Val Tyr Tyr Arg Pro Val Asp Gln Tyr Ser
   145                 150                 155                 160
   aac cag aac aac ttc gtg cac gac tgc gtc aat atc acc atc aag cag     528
   Asn Gln Asn Asn Phe Val His Asp Cys Val Asn Ile Thr Ile Lys Gln
                   165                 170                 175
   cac acg gtc acc acc acc acc aag ggg gag aac ttc acc gag acc gat     576
   His Thr Val Thr Thr Thr Thr Lys Gly Glu Asn Phe Thr Glu Thr Asp
               180                 185                 190
   gtg aag atg atg gag cgc gtg gtg gag cag atg tgc gtc acc cag tac     624
   Val Lys Met Met Glu Arg Val Val Glu Gln Met Cys Val Thr Gln Tyr
           195                 200                 205
   cag aag gag tcc cag gcc tat tac gac ggg aga aga tcc agc gct gga     672
   Gln Lys Glu Ser Gln Ala Tyr Tyr Asp Gly Arg Arg Ser Ser Ala Gly
       210                 215                 220
   gcg atc ggt gga gct aaa aag cgg cca aag cct gga ggg tgg aac act     720
   Ala Ile Gly Gly Ala Lys Lys Arg Pro Lys Pro Gly Gly Trp Asn Thr
   225                 230                 235                 240
   ggc gga agc cga tac cct ggg cag ggc agc cct gga ggc aac cgt tac     768
   Gly Gly Ser Arg Tyr Pro Gly Gln Gly Ser Pro Gly Gly Asn Arg Tyr
                   245                 250                 255
   cca cct cag ggt ggc acc tgg ggg cag ccc cac ggt ggt ggc tgg gga     816
   Pro Pro Gln Gly Gly Thr Trp Gly Gln Pro His Gly Gly Gly Trp Gly
               260                 265                 270
   caa ccc cat ggg ggc agc tgg gga caa cct cat ggt ggt agt tgg ggt     864
   Gln Pro His Gly Gly Ser Trp Gly Gln Pro His Gly Gly Ser Trp Gly
           275                 280                 285
   cag ccc cat ggc ggt gga tgg ggc caa gga ggg ggt acc cac aat cag     912
   Gln Pro His Gly Gly Gly Trp Gly Gln Gly Gly Gly Thr His Asn Gln
       290                 295                 300
   tgg aac aag ccc aat aag cca aaa acc aac atg aag cac atg gcc ggc     960
   Trp Asn Lys Pro Asn Lys Pro Lys Thr Asn Met Lys His Met Ala Gly
   305                 310                 315                 320
   gct gct gcg gca ggg gcc gtg gtg ggg ggc ctt ggt ggc tac atg ctg    1008
   Ala Ala Ala Ala Gly Ala Val Val Gly Gly Leu Gly Gly Tyr Met Leu
                   325                 330                 335
   ggg agc gcc atg agc agg ccc atg atc cat ttt ggc aac gac tgg gag    1056
   Gly Ser Ala Met Ser Arg Pro Met Ile His Phe Gly Asn Asp Trp Glu
               340                 345                 350
   gac cgc tac tac cgt gaa aac atg tac cgc tac cct aac caa gtg tac    1104
   Asp Arg Tyr Tyr Arg Glu Asn Met Tyr Arg Tyr Pro Asn Gln Val Tyr
           355                 360                 365
   tac agg cca gtg gat cag tac agc aac cag aac aac ttc gtg cac gac    1152
   Tyr Arg Pro Val Asp Gln Tyr Ser Asn Gln Asn Asn Phe Val His Asp
       370                 375                 380
   tgc gtc aat atc acc atc aag cag cac acg gtc acc acc acc acc aag    1200
   Cys Val Asn Ile Thr Ile Lys Gln His Thr Val Thr Thr Thr Thr Lys
   385                 390                 395                 400
   ggg gag aac ttc acc gag acc gat gtg aag atg atg gag cgc gtg gtg    1248
   Gly Glu Asn Phe Thr Glu Thr Asp Val Lys Met Met Glu Arg Val Val
                   405                     410                 415
   gag cag atg tgc gtc acc cag tac cag aag gag tcc cag gcc tat tac    1296
   Glu Gln Met Cys Val Thr Gln Tyr Gln Lys Glu Ser Gln Ala Tyr Tyr
               420                 425                 430
   gac ggg aga aga tcc agc agc tag                                    1320
   Asp Gly Arg Arg Ser Ser Ser
           435
<210> 2
   <211> 439
   <212> PRT
   <213> Artificial Sequence
<220>
   <223> Description of Artificial Sequence:
   fusion protein
<400> 2
   Met Arg Gly Ser His His His His His His Gly Ser Cys Lys Lys Arg
    1               5                  10                  15
   Pro Lys Pro Gly Gly Trp Asn Thr Gly Gly Ser Arg Tyr Pro Gly Gln
               20                  25                  30
   Gly Ser Pro Gly Gly Asn Arg Tyr Pro Pro Gln Gly Gly Thr Trp Gly
           35                  40                  45
   Gln Pro His Gly Gly Gly Trp Gly Gln Pro His Gly Gly Ser Trp Gly
       50                  55                  60
   Gln Pro His Gly Gly Ser Trp Gly Gln Pro His Gly Gly Gly Trp Gly
   65                  70                  75                  80
   Gln Gly Gly Gly Thr His Asn Gln Trp Asn Lys Pro Ser Lys Pro Lys
                   85                  90                  95
   Thr Asn Met Lys His Met Ala Gly Ala Ala Ala Ala Gly Ala Val Val
               100                 105                 110
   Gly Gly Leu Gly Gly Tyr Met Leu Gly Ser Ala Met Ser Arg Pro Met
           115                 120                 125
   Ile His Phe Gly Asn Asp Trp Glu Asp Arg Tyr Tyr Arg Glu Asn Met
       130                 135                 140
   Tyr Arg Tyr Pro Asn Gln Val Tyr Tyr Arg Pro Val Asp Gln Tyr Ser
   145                 150                 155                 160
   Asn Gln Asn Asn Phe Val His Asp Cys Val Asn Ile Thr Ile Lys Gln
                   165                 170                 175
   His Thr Val Thr Thr Thr Thr Lys Gly Glu Asn Phe Thr Glu Thr Asp
               180                 185                 190
   Val Lys Met Met Glu Arg Val Val Glu Gln Met Cys Val Thr Gln Tyr
           195                 200                 205
   Gln Lys Glu Ser Gln Ala Tyr Tyr Asp Gly Arg Arg Ser Ser Ala Gly
       210                 215                 220
   Ala Ile Gly Gly Ala Lys Lys Arg Pro Lys Pro Gly Gly Trp Asn Thr
   225                 230                 235                 240
   Gly Gly Ser Arg Tyr Pro Gly Gln Gly Ser Pro Gly Gly Asn Arg Tyr
                   245                 250                 255
   Pro Pro Gln Gly Gly Thr Trp Gly Gln Pro His Gly Gly Gly Trp Gly
               260                 265                 270
   Gln Pro His Gly Gly Ser Trp Gly Gln Pro His Gly Gly Ser Trp Gly
           275                 280                 285
   Gln Pro His Gly Gly Gly Trp Gly Gln Gly Gly Gly Thr His Asn Gln
       290                 295                 300
   Trp Asn Lys Pro Asn Lys Pro Lys Thr Asn Met Lys His Met Ala Gly
   305                 310                 315                 320
   Ala Ala Ala Ala Gly Ala Val Val Gly Gly Leu Gly Gly Tyr Met Leu
                   325                 330                 335
   Gly Ser Ala Met Ser Arg Pro Met Ile His Phe Gly Asn Asp Trp Glu
               340                 345                 350
   Asp Arg Tyr Tyr Arg Glu Asn Met Tyr Arg Tyr Pro Asn Gln Val Tyr
           355                 360                 365
   Tyr Arg Pro Val Asp Gln Tyr Ser Asn Gln Asn Asn Phe Val His Asp
       370                 375                 380
   Cys Val Asn Ile Thr Ile Lys Gln His Thr Val Thr Thr Thr Thr Lys
   385                 390                 395                 400
   Gly Glu Asn Phe Thr Glu Thr Asp Val Lys Met Met Glu Arg Val Val
                   405                 410                 415
   Glu Gln Met Cys Val Thr Gln Tyr Gln Lys Glu Ser Gln Ala Tyr Tyr
               420                 425                 430
   Asp Gly Arg Arg Ser Ser Ser
           435
<210> 3
   <211> 7
   <212> PRT
   <213> Artificial Sequence
<220>
   <223> Description of Artificial Sequence:
   peptide spacer
<400> 3
   Ala Gly Ala Ile Gly Gly Ala
                   5
<210> 4
   <211> 6
   <212> PRT
   <213> Artificial Sequence
<220>
   <223> Description of Artificial Sequence:
   peptide spacer
<400> 4
   Ser Gly Gly Arg Gly Gly
                   5
<210> 5
   <211> 17
   <212> PRT
   <213> Artificial Sequence
<220>
   <223> Description of Artificial Sequence:
   peptide spacer
<400> 5
   Gly Ser Gly Gly Gly Gly Ser Gly Gly Gly Gly Ser Gly Gly Ser Gly
                   5                   10                  15
   Ala
<210> 6
   <211> 5
   <212> PRT
   <213> Artificial Sequence
<220>
   <223> Description of Artificial Sequence:
   peptide spacer
<400> 6
   Gly Gly Gly Gly Ser
                   5
<210> 7
   <211> 10
   <212> PRT
   <213> Artificial Sequence
<220>
   <223> Description of Artificial Sequence:
   peptide spacer
<400> 7
   Gly Gly Gly Gly Ser Gly Gly Gly Gly Ser
   5 10
<210> 8
   <211> 15
   <212> PRT
   <213> Artificial Sequence
<220>
   <223> Description of Artificial Sequence:
   peptide spacer
<400> 8
   Gly Gly Gly Gly Ser Gly Gly Gly Gly Ser Gly Gly Gly Gly Ser
   5 10 15
<210> 9
   <211> 7
   <212> PRT
   <213> Artificial Sequence
<220>
   <223> Description of Artificial Sequence:
   peptide spacer
<400> 9
   Ala Gly Ala Ile Gly Gly Ala
   5
<210> 10
   <211> 34
   <212> DNA
   <213> Artificial Sequence
<220>
   <223> Description of Artificial Sequence:
   Primer
<400> 10
   gcggatccgt cgccaccatg gcgaaccttg gcta 34
<210> 11
   <211> 38
   <212> DNA
   <213> Artificial Sequence
<220>
   <223> Description of Artificial Sequence:
   Primer
<400> 11
   accgatcgct ccagcgctgg atcttctccc gtcgtaat 38
<210> 12
   <211> 38
   <212> DNA
   <213> Artificial Sequence
<220>
   <223> Description of Artificial Sequence:
   Primer
<400> 12
   agcgatcggt ggagctaaaa agcggccaaa gcctggag 38
<210> 13
   <211> 33
   <212> DNA
   <213> Artificial Sequence
<220>
   <223> Description of Artificial Sequence:
   Primer
<400> 13
   atctagatca tcatcatccc acgatcagga aga 33
<210> 14
   <211> 30
   <212> DNA
   <213> Artificial Sequence
<220>
   <223> Description of Artificial Sequence:
   Primer
<400> 14
   gatgttggat cctgcaagaa gcggccaaag 30
<210> 15
   <211> 29
   <212> DNA
   <213> Artificial Sequence
<220>
   <223> Description of Artificial Sequence:
   Primer
<400> 15
   ggaggagatc cagcagctag aagcttttc 29

## Description

The present invention relates to a prion protein, wherein said prion protein is a homodimer or heterodimer. The prion protein dimers are highly immunogenic capable of inducing an immune response in a mammal, thus useful for prophylactic or therapeutic vaccination against diseases associated with the infectious forms of prion proteins, PrP^{sc}, i.e. transmissible spongiform encephalopathies. Furthermore, the present invention relates to antibodies generated by using the prion protein dimers as an antigen, pharmaceutical compositions containing the prion protein dimers or antibodies directed against said dimers as well as DNA sequences encoding the prion protein dimers.

Transmissible spongiform encephalopathies are neurodegenerative diseases such as scrapie of sheep, bovine spongiform encephalopathy (BSE) of cattle and Creutzfeldt-Jakob disease (CJD) of man. Infectious preparations derived from infected brains are resistant to ultraviolet and ionizing radiation as well as other procedures which normally inactivate nucleic acids indicating that nucleic acids are not required for infectivity. Purification of infectious preparations from brains revealed the presence of a protein required for infectivity. These experimental observations led to the "protein only" hypothesis which proposes that particular proteinaceous infectious particles (prions) are responsible for the transmission of transmissible spongiform encephalopathies (Prusiner et al., Proc. Natl. Acad. Sci. USA 95, 13363-13383 (1998)). Prions consist mainly of a protease resistent protein designated PrP^{sc} (prion protein, "Sc": scrapie), which is an abnormal isoform of the proteinase K sensitive PrP^{c} ("C":cellular). The prion protein PrP^{c} is a species specific protein which is physiologically expressed in brain cells and peripheral blood cells as well as, e.g., in spleen. The biological function of PrP^{c} is so far unknown. Transgenic mice which are no longer capable of expressing PrP^{c} (PrP knock out mice) do not show any pathological damages and cannot be infected with prions (Bueler et al., Nature 356, 577-582 (1992); Bueler et al., Cell 73, 1339-1347 (1993)).

Both isoforms, PrP^{sc} and PrP^{c}, share the same amino acid sequence, but differ in their secondary structure. Circular Dichroism (CD) and Fourier Transform Infrared (FTIR) spectroscopy revealed a significantly higher β-sheet content for PrP^{Sc} as compared to a high α-helix content in PrP^{c}. It has been suggested that prion propagation involves the conversion of α-helical domains in PrP^{c} into β-sheets in PrP^{Sc}. The in vitro conversion of PrP^{c} into a PrP^{Sc}-like molecule was demonstrated employing a proteinase K resistance assay.

It has been suggested that for the development of a transmissible spongiform encephalopathy the interaction of PrP^{sc} and PrP^{c} is a crucial event, wherein PrP^{Sc} forces a conversion of PrP^{c} into the pathological conformation (PrP^{Sc}) . Accordingly, transgenic mice which are no longer capable of expressing PrP^{c} (PrP knock out mice) are resistant to infections with PrP^{Sc}. Although, the prion proteins of various species differ as regards their amino acid sequences, within the group of mammals there is a high homology (88 to 99%). Apparently, due to differences in the amino acid sequences the prion protein cannot be transmitted between certain species (relative or absolute species barrier). Due to the fact that the prion protein is a physiologically expressed protein, each species exhibits immunological tolerance via its own specific prion protein. Unfortunately, so far transmissible spongiform encephalopathies, e.g. BSE, are incurable. There is no therapy available for soothing the symptoms of the disease, let alone for stopping the disease, e.g. by vaccination.

Priola S.A. et al. ((1995) J. Biol Chem.; vol. 270, no. 7, 3299-3305) disclose a 60-kDa prion protein from hamster which appeared as a dimer of covalently coupled PrP monomers.

Therefore, it is the object of the present invention to provide a vaccine for the prevention or treatment of a transmissible spongiform encephalopathy.

According to the invention this is achieved by the subject matters defined in the claims.

The present invention provides a prion protein, wherein said prion protein is a homodimer or heterodimer with the monomer corresponding to or comprising a prion protein according to the definition below. During the experiments leading to the present invention, it has, surprisingly, been found that prion protein dimers are useful for inducing an enhanced immune response against PrP^{c} and PrP^{Sc}. Using this apparent remarkable immunogenicity of the prion protein dimer it was even possible in vivo to generate antibodies directed against the homologous prion protein (i.e. mouse antibodies directed against murine PrP), thereby abolishing or by-passing the known auto-tolerance present within a given species. After incubation of cells permanently producing PrP^{Sc} with the antibodies that were generated by immunization with the prion protein dimer for 16 hours a drastic inhibition of the *de novo* synthesis of PrP^{sc} could be observed. Presumably, this result is due to generation of a functional PrP^{c} knock out state where the surface-located PrP^{c} substrate needed for continuous cellular conversion into PrP^{Sc} is functionally impaired, resulting finally in inhibition of generation of PrP^{sc}. It is known from the literature that the surface location of PrP^{c} is a prerequisite for cellular prion conversion (Taraboulos et al., J. Cell Biol. 129, 121-132 (1995)). Even more, studies with transgenic mice have demonstrated that peripheral expression of PrP^{c} is absolutely required for replication and transport of PrP^{sc} from peripheral sites of the body to the central nervous system (Blättler et al., Nature 389, 69-73 (1997)). In this respect it has been discovered by the inventors that after application of the prion protein dimer of the present application to a subject, the induced immune response results in the downregulation of the surface expression of PrP^{c}. Accordingly, the conversion rate (PrP^{c}⇒PrP^{sc}) can be drastically reduced, thus, blocking the initiation and the progression of the disease. Thus, the prion protein dimers of the present invention are useful as a vaccine for the prevention or treatment of a transmissible spongiform encephalopathy.

Accordingly, the present invention relates to a prion protein, wherein said prion protein is a homodimer or heterodimer which can elicit an immune response against PrP^{c} or PrP^{sc}, preferably an immune response which is higher compared to the immune response obtained using for vaccination the corresponding monomer. As used herein, the term "prion protein" comprises the native (full length and/or mature) protein as well as variants thereof, e.g. truncated versions, which are still useful for eliciting an enhanced immune response, i.e. still useful for vaccination. In this context, the term "protein" also comprises peptides or polypeptides having a length of at least 8, preferably of at least 15 and, more preferably, of at least 20 amino acids. The person skilled in the art knows sources for the prion protein monomers and DNA sequences encoding the prion protein monomers, respectively (Schätzl et al., J. Mol. Biol. 245, 362-374 (1995); Wopfner et al., J. Mol. Biol. 289, 1163-1178 (1999); sequences deposited at Genbank accessible via Public Medline database). Furthermore, a person skilled in the art knows methods for coupling the monomers in order to obtain the dimer. The monomers are covalently coupled.

In a preferred embodiment, the homodimer or heterodimer is a fusion protein comprising the monomeric prion protein units. Such a fusion protein can be generated by methods well known by the person skilled in the art. The selection of a particular method mainly depends on the length of the monomers, i.e. very short fusion proteins are preferably chemically synthesized, e.g. by standard solid phase synthesis, whereas longer fusion proteins are preferably produced by expression of the corresponding DNA sequences in a suitable host and isolation/purification of the fusion protein from the host or the culture medium.

Generally, by means of conventional molecular biological processes (see, e.g., Sambrook et al., 1989, Molecular Cloning, A Laboratory Manual, 2^{nd} edition Cold Spring Harbor Laboratory Press, Cold Spring Harbor, NY) it is not only possible to generate a DNA sequence encoding the desired fusion protein but, additionally, to introduce different mutations into the DNA sequence. One possibility is the production of deletion mutants in which DNA molecules are produced by continuous deletions from the 5'- or 3'-termini of the coding DNA sequence and that lead to the synthesis of proteins that are shortened accordingly. The person skilled in the art can easily check as to whether such prion protein dimers containing shortened monomeric units are still useful as a vaccine, e.g. using the methods described in the examples, below. For the manipulation in prokaryotic cells by means of genetic engineering the DNA sequences can be introduced into plasmids allowing a mutagenesis or a modification of a sequence by recombination of DNA sequences. By means of conventional methods (cf. Sambrook et al., 1989, Molecular Cloning: A Laboratory Manual, 2^{nd} edition, Cold Spring Harbor Laboratory Press, NY, USA) bases can be exchanged and natural or synthetic sequences can be added. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

In a more preferred embodiment, the monomers of the fusion protein are linked via a peptide spacer. Suitable peptide spacers, which preferably consist of non-polar and non-bulky amino acids, have a length below 20 residues, provide a certain flexibility, thereby avoiding e.g. cystein or prolin residues which can induce rigidity (or vice versa in cases when rigidity or induction of an orientation/turns are wanted). These spacers are known to the person skilled in the art and comprise for example AGAIGGA [HIV-1 protease; Kräusslich et al., Proc. Natl. Acad. Sci. USA, 88, 3212-3217 (1991)], SGGRGG [HIV gp41; Tan et al., Proc. Natl. Acad. Sci USA, 94, 12303-12308 (1991)], GSGGGGSGGGGSGGSGA [single chain T-cell receptor; Khandekar et al., J. Biol. Chem. 272, 32190-32197 (1997)] or (GGGGS)ₙ₌₁₋₃ [single chain antibodies; Kortt et al., Eur. J. Biochem. 221, 151-157 (1994)]. Examples of preferred spacers are (a) spacers having a length of 2 to 3 amino acids, wherein the DNA sequence encoding said spacer contains a restriction site, (b) oligomers composed of small neutral amino acids (e.g. Ala-Gly-Ala-Ile-Gly-Gly-Ala), or c) an oligomer having an amino acid sequence that defines an epitope, e.g. flag-tag, HA-epitope etc..

For the generation of the prion protein dimers encoding DNA sequences and for constructing expression vectors which contain said DNA sequences, it is possible to use general methods known in the art. These methods include e.g. in vitro recombination techniques, synthetic methods and in vivo recombination methods as described in Sambrook et al., Molecular Cloning, A Laboratory Manual, 2^{nd} edition (1989) Cold Spring Harbor Laboratory Press, Cold Spring Harbor, NY, for example.

For recombinant production of the dimeric prion protein of the invention, the DNA sequences encoding said protein are inserted into a suitable expression vector. Preferably, they are plasmids, cosmids, viruses, bacteriophages and other expression vectors usually used in the field of genetic engineering. Expression vectors suitable for use in the present invention include, but are not limited to e.g. pGEMEX, pUC derivatives, pGEX-2T, pET3b, T7 based expression vectors and pQE and pTrcHis (for expression in E.coli). For the expression in yeast, e.g. pY100 and Ycpad1 have to be mentioned while e.g. pcDNA3.1, pKCR, pEFBOS, cDM8, pMSCND, and pCEV4 have to be indicated for the expression in animal cells. The baculovirus expression vector pAcSGHisNT-A is especially suitable for the expression in insect cells. The DNA sequences can also be contained in a recombinant virus containing appropriate expression cassettes. Suitable viruses that may be used in the present invention include baculovirus, SFV, vaccinia, sindbis virus, SV40, Sendai virus, adenovirus, an AAV virus or a parvovirus, such as MVM or H-1. The vector may also be a retrovirus, such as MoMULV, MoMuLV, HaMuSV, MuMTV, RSV or GaLV. Preferably, the DNA sequence is operatively linked to the regulatory elements in the recombinant vector that guarantee the transcription and synthesis of an RNA in prokaryotic and/or eukaryotic cells that can be translated. The nucleotide sequence to be transcribed can be operably linked to a promoter like a T7, metallothionein I or polyhedrin promoter. For expression in mammals, the DNA sequences are operatively linked to a suitable promoter like, e.g. a human cytomegalovirus "immediate early promoter" (pCMV).

Suitable host cells include bacteria, yeast, insect and animal cells, preferably mammalian cells. The E. coli strains HB101, DH1, x1776, JM101, JM109, BL21, XL1Blue and SG 13009, the yeast strain Saccharomyces cerevisiae, the insect cells sf9 and the animal cells L, A9, 3T3, FM3A, BHK, human SW13, CHO, COS, Vero and HeLa are preferred. Methods of transforming these host cells, of phenotypically selecting transformants and of expressing the DNA according to the invention by using the above described vectors are known in the art. For recombinant production of the dimeric prion protein of the invention, the host cell is cultivated under conditions allowing the synthesis of the protein and the protein is subsequently isolated from the cultivated cells and/or the culture medium. Isolation and purification of the recombinantly produced proteins may be carried out by conventional means including preparative chromatography and immunological separations involving affinity chromatography with monoclonal or polyclonal antibodies, e.g. with the antibody A7 and 3F4 described in the examples, below.

In an alternative preferred embodiment the monomers of the dimeric prion protein of the invention are covalently coupled via suitable compounds, e.g. in such a way that the monomers are covalently linked via their C-termini or N-termini or via the C-terminus of one monomer and the N-terminus of the other monomer. Care has to be taken that dimerization is carried out in such a way that eliciting an immune response by the dimeric protein is not adversely effected. Suitable compounds for dimerization are known to the person skilled in the art, preferably such a compound is a polyethylenglycole, activated benzodiazepine, oxazolone, aminimide, azalactone, diketopiperazine or a monosaccharide. The corresponding chemical reactions are also known to the person skilled in the art.

The monomers of the dimeric prion protein of the present invention can be identical (homodimer: the monomers are derived from the same species) or different (heterodimer: the monomers are derived from different species). In the case of the heterodimer it can be expected that an immune response against the own PrP^{c} as well as the foreign PrP^{c} is induced.

As already described above the dimeric prion protein of the present invention also includes monomers which are variants of the native prion protein monomers. Particularly preferred are variants which are N-terminally and/or C-terminally truncated proteins (monomers).

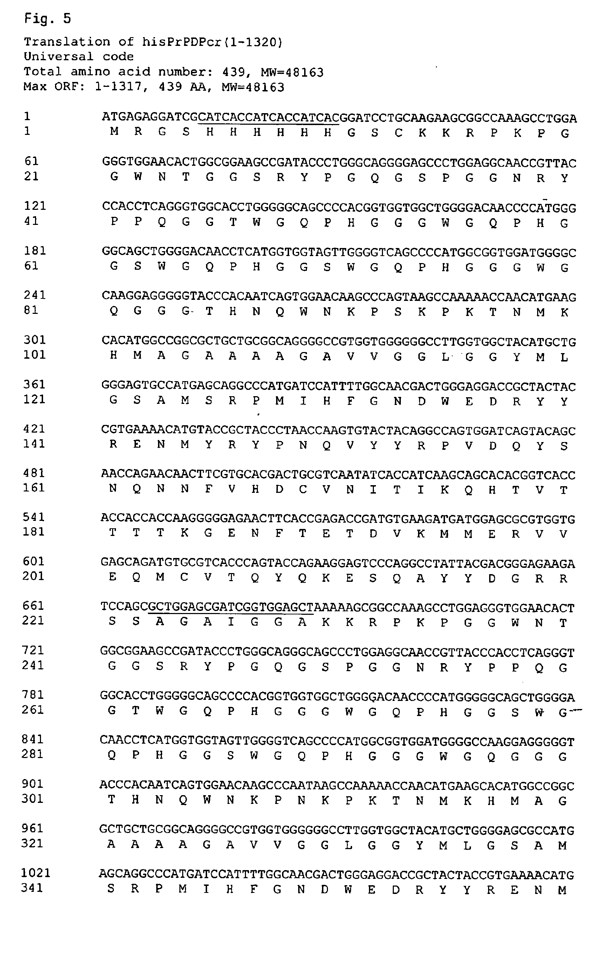

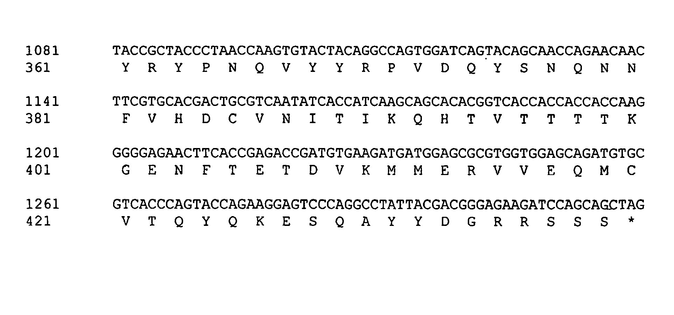

A preferred dimeric prion protein (and its corresponding nucleotide sequence) is shown in Fig. 5.

The present invention also relates to the use of a dimeric prion protein of the present invention for the production of polyclonal or monoclonal antibodies as well as to an antibody, preferably a monoclonal antibody which binds to the dimeric prion protein of the present invention. Such antibodies are useful for passive immunization and can be produced according to well known methods. The term "antibody", preferably, relates to antibodies which consist essentially of pooled monoclonal antibodies with different epitopic specificities, as well as distinct monoclonal antibody preparations. Monoclonal antibodies are made using as an antigen the dimeric prion protein of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain (scFv), and humanized antibodies.

Moreover, the present invention also relates to DNA sequences encoding the dimeric prion proteins of the present invention as well as an expression vector containing such a DNA sequence. Examples of such DNA sequences and suitable expression vectors are already described above. Such DNA sequences or expression vectors are not only useful for the recombinant production of the dimeric prion protein of the present invention but also useful as "genetic vaccine". For the preparation of a genetic vaccine, the DNA sequences of the invention are inserted into an appropriate vector under the control of a suitable promoter, e.g. a vector or promoter as described above. Additional suitable vectors are, e.g., herpes simplex virus type 1 amplicon or recombinant Semliki forest virus vectors, plus-strand RNA virus vectors (lentiviral vectors), minus-strand RNA virus vectors (e.g. Rabies virus, RSV). In this connection also "gene gun" applications are suitable. A vaccine according to the present invention can be used to stimulate humoral and/or cellular immune response in subjects who may benefit from such responses by protection against or treatment of a transmissible spongiform encephalopathy.

Finally, the present invention relates to a pharmaceutical composition comprising a dimeric prion protein, an antibody, DNA sequence or an expression vector of the present invention in a pharmaceutically acceptable carrier. After application to the subject, the immune response induced should result in the downregulation of the surface expression of PrP^{c}. Accordingly, the transport of infectious prions from peripheral sites of inoculation (e.g. oral or i.v.) to the central nervous system as well as the initial peripheral replication should be significantly impaired. In addition, the conversion rate (PrP^{c}⇒PrP^{Sc}) can be drastically reduced, thus blocking the progression of the disease. Thus, the compounds of the present invention are useful for the preparation of a vaccine for the prevention or treatment of a transmissible spongiform encephalopathy (prophylactic or therapeutic vaccination). For the preparation of the vaccine the purified compounds described above may be combined with any suitable adjuvant such as ISCOMS, alum, Freund's Incomplete or Complete Adjuvant, Quil A and other saponins or any other adjuvant as described in the literature. Suitable doses and routes of administration depend on the particular compound and are well known to the person skilled in the art.

### Brief description of the drawings

### Figure 1: PrP dimer

Coomassie blue staining after purification showing a purity of > 90%. M, molecular weight standards.

### Figure 2: Characterization of the PrP dimer using CD

The results show that the PrP monomer and the PrP dimer have a similar or even identical secondary structure.

### Figure 3

### (a) Immunological responses in different mice

Immunization with the PrP monomer and PrP dimer, respectively. Recombinant murine PrP was separated on a SDS PAGE gel, subsequently transferred to a PVDF membrane and the immunoblots were probed with decreasing concentrations auf mice antisera. The immunization with the PrP dimer resulted in an enhanced antibody production.

### (b) Immunological responses in different rabbits

Immunization with the PrP monomer and PrP dimer, respectively. Recombinant PrP monomer (PrP_{M}) and PrP dimer (PrP_{D}), respectively, were separated on a SDS PAGE gel, subsequently transferred to a PVDF membrane and the immunoblots were probed with anti-PrPₘₒₙₒₘₑᵣ and anti-PrP_{dimer}, respectively. The immunization with the PrP dimer resulted in an enhanced antibody production.

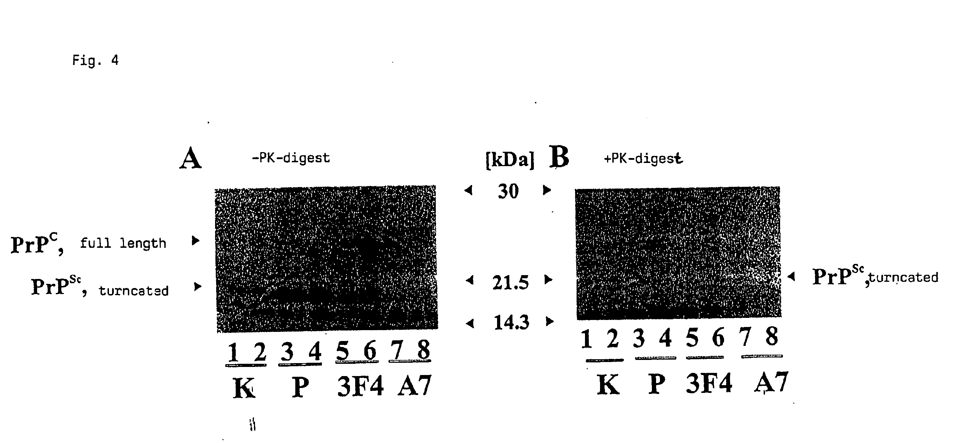

### Figure 4: In vitro experiments using PrP^{Sc} infected cells

During incubation with antibodies for 16 hours proteins were metabolically labeled using ³⁵S-methionine and subsequently immunoprecipitated (RIPA). Prior to the RIPA the cell lysates were separated into two fractions for digestion with proteinase K (+/-) and separated into soluble and insoluble fractions using ultracentrifugation (1 hour, 100,000 x g, 1% sarcosyl); data only shown for the insoluble fraction. Only after incubation with A7 a drastic decrease of the *de novo* synthesis of PrP^{Sc} can be observed. A,B: after deglycosylation using PNGase F; K: without incubation with antibodies; P: preimmune serum; 3F4: monoclonal antibody directed against the 3F4 epitope of the prion protein; A7: antibody obtained from rabbits which had been immunized with the PrP dimer.

### Figure 5: Translation of hisPrPDPcr (1-1320)

Underlined in the first line: His-Tag
Underlined in the middle: Linker

The present invention is explained by the examples.

### Example 1

### Generation of a fusion protein (dimer) comprising two prion protein monomers covalently linked by a peptide linker

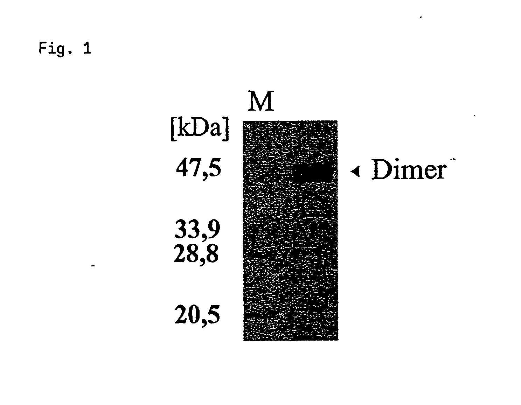

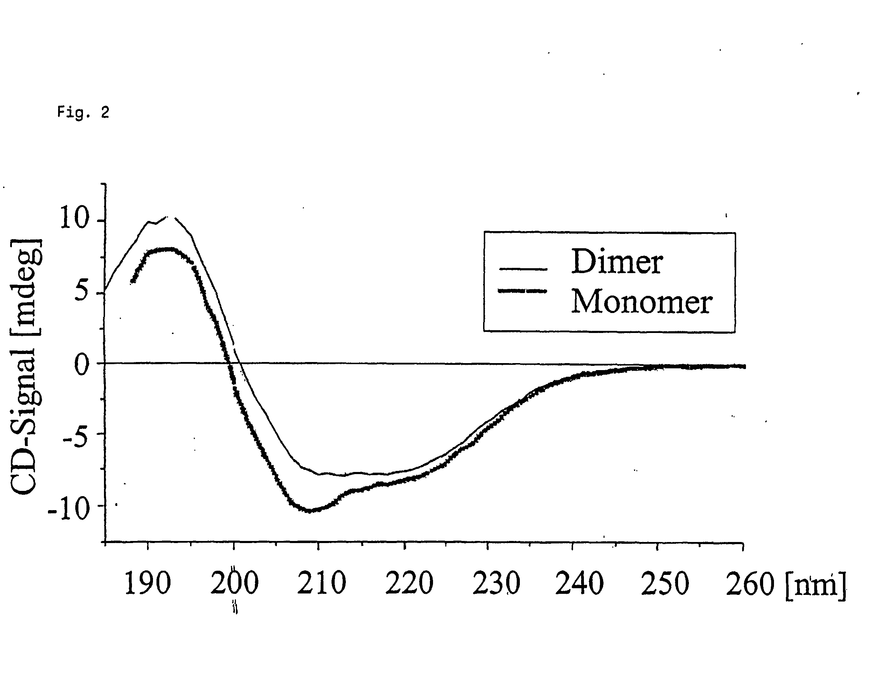

Mouse genomic DNA encoding the prion protein (murine PrP-A, derived from murine N2a neuroblastoma cell line; for sequence see Schätzl et al, 1995, supra; Wopfner et al., 1999, supra) was amplified by polymerase chain reaction (PCR) using the following primer pairs which introduce a peptide linker (Ala-Gly-Ala-Ile-Gly-Gly-Ala) encoding sequence containing a PvuI restriction site; primer pair I: 5'-GCG GAT CCG TCG CCA CCA TGG CGA ACC TTG GCT A-3' (PrPB+1) and 5'-**ACC GAT CG**C TCC AGC GCT GGA TCT TCT CCC GTC GTA AT-3' (231+L new); primer pair II: 5'-AG**C GAT CG**G TGG AGC TAA AAA GCG GCC AAA GCC TGG AG-3' (23+L new); 5'-ATC TAG ATC ATC ATC ATC CCA CGA TCA GGA AGA-3' (254 PX) (the DNA sequence encoding the PvuI restriction site is underlined). For the introduction of an N-terminal His-tag, the removal of the N-terminal and C-terminal signal peptides and the introduction of additional restriction sites, a second PCR was carried out using the following primers: 5'-GAT GTT **GGA TCC** TGC AAG AAG CGG CCA AAG-3' (5'-primer, the BamHI-site is underlined.); 5'-GGA GGA GAT CCA GCA GCT AG**A AGC TT**T TC-3' (3'-primer, the HindIII-site is underlined.) The PCR fragment obtained (c.f. Fig. 5) was inserted into the pQE30 expression vector (Qiagen, Hilden, DE). In order to obtain higher yields of the recombinantly produced protein, proteinase deficient bacteria (BL21, Stratagene Europe, Amsterdam, The Netherlands) were used for expression. Four hours after induction of expression using 2 mM IPTG, bacteria were lysed in 6 M guanidine hydrochloride (6 M guanidine hydrochloride, 20 mM sodium phosphate, 500 mM sodium chloride, pH 7,8). cell debris were pelleted by centrifugation (20 min., 10,000 x g), the supernatant was loaded on a Ni²⁺loaded SP-HiTrap column (Amersham Pharmacia, Uppsala, Sweden) which had been previously equilibrated using binding buffer (8 M urea, 20 mM sodium phosphate, 500 mM sodium chloride, pH 7.8) and incubated. The column was washed for several times (8 M urea, 20 mM sodium phosphate, 500 mM sodium chloride, 80 mM imidazole, pH 6.3) and, subsequently, the fusion protein containing the His-tag was eluted using 8 M urea, 20 mM sodium phosphate, 500 mM sodium chloride, 500 mM imidazole, pH 6.3. Fractions of the eluate were collected. The fractions showing the highest protein concentration were pooled and for removal of urea dialyzed against pure water (Slide-A-Lyzer, Pierce, Bonn, DE). As shown in Figure 1, the protein could be obtained with a very high purity. As shown in Figure 2, the secondary structure of the purified renatured dimer is similar (or even identical) to the secondary structure of the prion protein monomer.

### Example 2

### Immunization of mice and rabbits

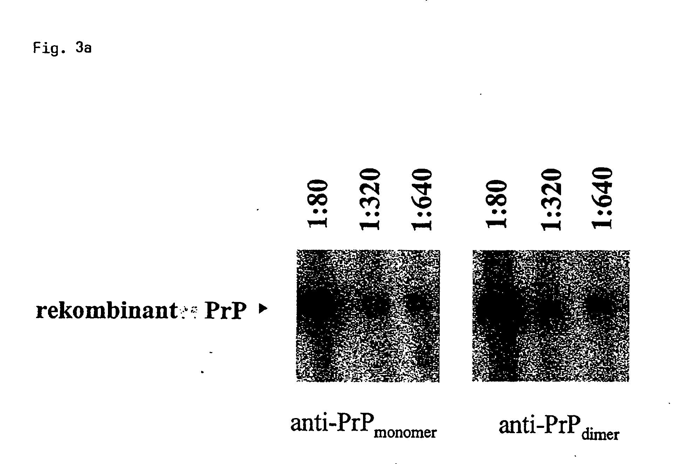

Ten female B6D₂F₁ (B57BL/6 x DBA) mice (inbred strain generated at the Institute of Molecular Animal Breeding/Gene Center Munich, DE; six weeks old) were immunized by subcutaneous injections of 50 µg of the protein dimer described in Example 1 and a control monomer, respectively (monomer: murine PrP23-231 verus dimer: murine PrP23-231=23-231 [connected by the linker sequence], both with the 3F4 epitope and an N-terminal poly-histidine tag). At day 0 the proteins were combined with Freund's Complete Adjuvant, for boostering (at day 21 and day 42, respectively) the proteins were combined with Freund's Incomplete Adjuvant. Ten days after the last immunization blood samples for antibody evaluation were taken. It could be shown that the application of the prion protein monomer (control) resulted in the generation of a lower amount of specific antibodies compared to the application of the prion protein dimer. After application of the dimer about 50% of mice showed a pronounced accumulation of specific antibodies (Figure 3a). The induced specific polyclonal immune response was measured in immunoblot. Recombinant murine PrP23-231 was separated on SDS-PAGE, transferred to PVDF membranes and incubated in an immunoblot analysis with serial dilutions of sera of immunized mice (primary antibody). The pre-immune sera were used to eliminate background signals. Staining was accomplished using conjugated secondary antibodies (antimouse) and the enhanced chemiluminescence kit (ECL plus, Amersham Corp., Buckinghamshire, U.K.). The mice immunized with PrP dimer showed higher antibody titers. Dilutions beyond 1:3000 still resulted in detectable signals. This finding indicates that the used immunogen was capable of overcoming the auto-tolerance phenomenon known for species-identical prion proteins. Further data showed that the induced antibodies were not directed against the N-terminal fusion domain or the linker sequence being reactive against authentic murine PrP23-231.

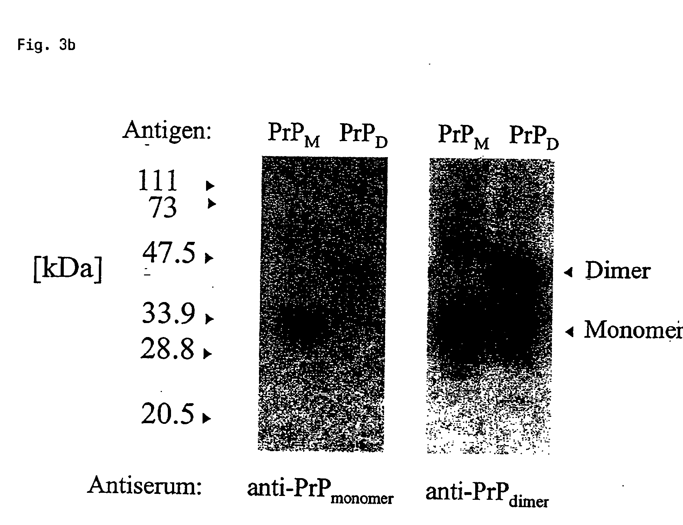

A similar result could already be achieved using rabbits, i.e. the rabbits immunized with the prion protein dimer showed a stronger immune response compared to the rabbits immunized with the prion protein monomer (Figure 3b). Recombinant monomeric and dimeric prion proteins (PrPM and PrPD, respectively, which were used also as immunogens in immunization) were separated on SDS-PAGE, transferred to PVDF membranes and analyzed in immunoblot. The left panel shows incubation with the serum from the rabbit immunized with PrP monomer, the right panel from the arbbit immunized with PrP dimer. Staining was accomplished using conjugated anti-rabbit secondary antibodies and the ECL plus kit. Whereas the left panel shows mainly a reaction against the monomeric PrP, the right panel demonstrates a much stronger immune reactivity which is directed against monomeric and dimeric prion protein.

### Example 3

### The application of antibodies generated by immunization with a prion protein dimer leads to inhibition of the de novo synthesis of PrP^{Sc} in vitro

The biological relevance of the immunization experiments described in Example 2 could be verified by the following in vitro experiment. ScMHM2 cells [cultured murine neuroblastoma cells (ATCC CCL 131); persistently infected with RML prions; stably transfected with a 3F4-tagged PrP; c.f. Schätzl et al., J. Virol. 71, 8821-8831, (1997)] which are infected with the infectious version of the prion protein (PrP^{Sc}) and permanently produce PrP^{Sc} were incubated with different types of antibodies. Incubation was carried using (a) commercially available monoclonal antibodies (3F4) (Signet Pathology Systems, Dedham, MA, USA) which are directed against an epitope of the prion protein produced in ScMHM2 cells and (b) the antiserum obtained from rabbits immunized with the prion protein dimer described in Example 1. During incubation with antibodies for 16 hours proteins were metabolically labeled using ³⁵S-methionine and subsequently immunoprecipitated (RIPA). Prior to the RIPA the cell lysates were separated into two fractions for digestion with proteinase K (+/-) and separated into soluble and insoluble fractions using ultracentrifugation (1 hour, 100,000 x g, 1% sarcosyl). As shown in Figure 4, after incubation with the antibodies for 16 hours a drastic inhibition of the *de novo* synthesis of PrP^{Sc} could be observed. The results observed with the 3FA antibody are less pronounced. It can be concluded that this result is due to generation of a functional PrP^{c} knock out state on the cell surface and, accordingly, an inhibition of the generation of PrP^{Sc}. These results demonstrate in living cells that the induced anti-PrP antibodies are capable of binding to surface-located authentic PrP^{c} under native conditions (proof of principle for interaction with surface-located PrP under native conditions). Even more, bound antibodies are apparently internalised with PrP^{c} demonstrating a high binding affinity (resulting in an insoluble PrP of full length which is PK sensitive, see full-length signal in Fig. 4A, lanes 5-8). Finally, endogeneous PrP^{Sc} biogenesis was heavily impaired resulting in no more de novo generated PK resistant PrP^{sc} (Fig. 4B, lanes 7 and 8). The biological effect on PrP^{sc} biogenesis is therefore demonstrated as well (proof of principle for impairment of prion biogenesis). The efficiency of this principle could also be shown by using particular chemical compounds: The functional removal of surface-PrP^{c} resulted in a reduction of the production of prion proteins.

## Claims

1. A prion protein, wherein said prion protein is:
(a) a heterodimer, comprising covalently coupled prion protein (PrP) monomers derived from different species; or
(b) a fusionprotein, wherein the fusionprotein is a homodimer comprising prion protein (PrP) monomers derived from the same species or a heterodimer comprising prion protein (PrP) monomers derived from different species; or
(c) a homodimer comprising prion protein (PrP) monomers derived from the same species or a heterodimer comprising prion protein (PrP) monomers derived from different species, wherein the prion protein(PrP) monomers are covalently coupled via polyethylenglycole, activated benzodiazepine, oxazolone, azalactone, diketopiperazine or a monosaccharide

2. The prion protein of claim 1(b), wherein the prion protein (PrP) monomers of the fusion protein are linked via a peptide spacer.

3. The prion protein of claim 2, wherein the peptide spacer is (a) a spacer having a length of 2 to 3 amino acids, wherein the DNA sequence encoding said spacer contains a restriction site or (b) an oligomer composed of small neutral amino acids or c) an oligomer having an amino acid sequence that defines an epitope.

4. The prion protein of any one of claims 1 to 3, wherein the monomers of the prion protein (PrP) dimer comprise the native prion protein or a variant thereof, wherein the variant of the native prion protein has a length of at least 8 amino acids and the prion protein dimer is useful for eliciting an immune response against PrP^{c} or PrP^{Sc} which is higher compared to the immune response obtained using the corresponding monomer for vaccination.

5. The prion protein of claim 4, wherein the variant is a N-terminally and/or C-terminally truncated prion protein.

6. Use of the a prion protein of any one of claims 1 to5. for the production of a polyclonal or monoclonal antibody.

7. An antibody which specifically binds to a prion protein of the same species according to any one of claims 1 to 5.

8. The antibody of claim 7 which is a monoclonal antibody.

9. A DNA sequence encoding a-fusionprotein of the prion protein of anyone of the claims 1b) and 2 to 5.

10. An expression vector containing a DNA sequence of claim 9.

11. A pharmaceutical composition containing a prion protein, wherein said prion protein is a homodimer comprising covalently coupled prion protein (PrP) monomers derived from the same species or a heterodimer comprising covalently coupled prion protein (PrP) monomers derived from different species, an antibody according to claim 7 or 8, a DNA sequence of claim 9 or an expression vector of claim 10.

12. Use of a prion protein, wherein said prion protein is a homodimer comprising covalently coupled prion protein (PrP) monomers derived from the same species or a heterodimer comprising covalently coupled prion protein (PrP) monomers derived from different species an antibody according to claim 7 or 8, a DNA sequence of claim 9 or an expression vector of claim 10 for the preparation of a vaccine for the prevention or treatment of a transmissible spongiform encephalopathy.

13. Use of claim 12, wherein the prion protein is a prion protein according to anyone of the claims 1 to 5.

## Patentansprüche

1. Prionprotein, wobei das Prionprotein ist:
(a) ein Heterodimer, umfassend kovalent gebundene Prionprotein(PrP)-Monomere von unterschiedlichen Spezies; oder
(b) ein Fusionsprotein, wobei das Fusionsprotein ein Homodimer, die Prionprotein(PrP)-Monomere von derselben Spezies umfasst, oder ein Heterodimer ist, das Prionprotein(PrP)-Monomere von unterschiedlichen Spezies umfasst; oder
(c) ein Homodimer, umfassend Prionprotein(PrP)-Monomere von derselben Spezies, oder ein Heterodimer, umfassend Prionprotein(PrP)-Monomere von unterschiedlichen Spezies, wobei die Prionprotein(PrP)-Monomere kovalent über Polyethylenglycol, aktiviertes Benzodiazepin, Oxazolon, Azalacton, Diketopiperazin oder Monosaccharid gebunden sind.

2. Prionprotein nach Anspruch 1(b), wobei die Prionprotein(PrP)-Monomere des Fusionsproteins über einen Peptid-Spacer gebunden sind.

3. Prionprotein nach Anspruch 2, wobei der Peptid-Spacer (a) ein Spacer mit einer Länge von 2 bis 3 Aminosäuren, wobei die DNA-Sequenz, die den Spacer kodiert, eine Restriktions-Schnittstelle enthält, oder (b) ein Oligomer, das aus kleinen neutralen Aminosäuren aufgebaut wird, oder c) ein Oligomer mit einer ein Epitop definierenden Aminosäuresequenz ist.

4. Prionprotein nach einem der Ansprüche 1 bis 3, wobei die Monomere des Prionprotein(PrP)-Dimeren das native Prionprotein oder eine Variante davon umfassen, wobei die Variante des nativen Prionproteins eine Länge von mindestens 8 Aminosäuren hat und das Prionproteindimer zum Hervorrufen einer Immunreaktion gegenüber PrP^{c} oder PrP^{sc} nützlich ist, die höher als die lmmunreaktion ist, die mittels des entsprechenden Monomers zur Impfung erhalten wird.

5. Prionprotein nach Anspruch 4, wobei die Variante ein N-terminal und/oder C-terminal verkürztes Prionprotein ist.

6. Verwendung des Prionproteins nach einem der Ansprüche 1 bis 5, zur Herstellung eines polyklonalen oder monoklonalen Antikörpers.

7. Antikörper der spezifisch an ein Prionprotein derselben Art nach einem der Ansprüche 1 bis 5 bindet.

8. Antikörper nach Anspruch 7, bei dem es sich um einen monoklonalen Antikörper handelt.

9. DNA-Sequenz, die für ein Fusionsprotein des Prionproteins nach einem der Ansprüche 1b) und 2 bis 5 kodiert.

10. Expressionsvektor, enthaltend eine DNA-Sequenz nach Anspruch 9.

11. Pharmazeutische Zusammensetzung, enthaltend ein Prionprotein, wobei das Prionprotein ein Homodimer, umfassend kovalent gebundene Prionprotein(PrP)-Monomere von derselben Spezies, oder ein Heterodimer ist, umfassend kovalent gebundene Prionprotein(PrP)-Monomere von unterschiedlichen Spezies, einen Antikörper nach Anspruch 7 oder 8, eine DNA-Sequenz nach Anspruch 9 oder einen Expressionsvektor nach Anspruch 10.

12. Verwendung eines Prionproteins, wobei das Prionprotein ein Homodimer, umfassend kovalent gebundene Prionprotein(PrP)-Monomere von derselben Spezies, oder ein Heterodimer ist, umfassend kovalent gebundene Prionprotein(PrP)-Monomere von unterschiedlichen Spezies, eines Antikörpers nach Anspruch 7 oder 8, einer DNA-Sequenz nach Anspruch 9 oder eines Expressionsvektors nach Anspruch 10 zur Herstellung eines Impfstoffs zur Verhinderung oder Behandlung einer übertragbaren spongiformen Enzephalopathie.

13. Verwendung nach Anspruch 12, wobei das Prionprotein ein Prionprotein nach einem der Ansprüche 1 bis 5 ist.

## Revendications

1. Protéine prion, **caractérisée en ce que** ladite protéine prion est :
(a) un hétérodimère comprenant des monomères de protéine prion (PrP) couplés de façon covalente, dérivés de différentes espèces, ou
(b) une protéine de fusion, où la protéine de fusion est un homodimère comprenant des monomères de protéine prion (PrP) dérivés de la même espèce ou un hétérodimère comprenant des monomères de protéine prion (PrP) dérivés de différentes espèces, ou
(c) un homodimère comprenant des monomères de protéine prion (PrP) dérivés de la même espèce ou un hétérodimère comprenant des monomères de protéine prion (PrP) dérivés d'espèces différentes, où les monomères de protéine prion (PrP) sont couplés de façon covalente via du polyéthylène glycol, de la benzodiazépine activée, de l'oxazolone, de l'azalactone, de la dikétopipérazine ou un monosaccharide.

2. Protéine prion de la revendication 1(b), **caractérisée en ce que** les monomères de protéine prion (PrP) de la protéine de fusion sont liés via un peptide intercalaire.

3. Protéine prion de la revendication 2, **caractérisée en ce que** l'intercalaire peptide est (a) un intercalaire ayant une longueur de 2 à 3 acides aminés, où la séquence d'ADN codant pour ledit intercalaire contient un site de restriction ou (b) un oligomère composé de petits acides aminés neutres ou (c) un oligomère ayant une séquence d'acides aminés qui définit un épitope.

4. Protéine prion selon l'une quelconque des revendications 1 à 3, **caractérisée en ce que** les monomères du dimère de protéine prion (PrP) comprennent la protéine prion native ou une variante de celle-ci, où la variante de la protéine prion native a une longueur d'au moins 8 acides aminés et le dimère de protéine prion est utile pour obtenir une réponse immunitaire contre la PrP^{c} ou la PrP^{sc} qui est plus importante que la réponse immunitaire obtenue en utilisant le monomère correspondant pour la vaccination.

5. Protéine prion de la revendication 4, **caractérisée en ce que** la variante est une protéine prion tronquée à sa terminaison N ou à sa terminaison C.

6. Utilisation de la protéine prion de l'une quelconque des revendications 1 à 5 pour la production d'un anticorps monoclonal ou polyclonal.

7. Anticorps qui se lie spécifiquement à une protéine prion de la même espèce selon l'une quelconque des revendications 1 à 5.

8. Anticorps de la revendication 7 qui est un anticorps monoclonal.

9. Séquence d'ADN codant pour une protéine de fusion de la protéine prion de l'une quelconque des revendications 1 et 2 à 5.

10. Vecteur d'expression contenant une séquence d'ADN de la revendication 9.

11. Composition pharmaceutique contenant une protéine prion, **caractérisée en ce que** ladite protéine prion est un homodimère comprenant des monomères de protéine prion (PrP) couplés de façon covalente, dérivés de la même espèce ou un hétérodimère comprenant des monomères de protéine prion (PrP) couplés de façon covalente, dérivés d'espèces différentes, un anticorps selon la revendication 7 ou 8, une séquence d'ADN selon la revendication 9 ou un vecteur d'expression selon la revendication 10.

12. Utilisation d'une protéine prion, **caractérisée en ce que** ladite protéine prion est un homodimère comprenant des monomères de protéine prion (PrP) couplés de façon covalente, dérivés de la même espèce ou un hétérodimère comprenant des monomères de protéine prion (PrP) couplés de façon covalente, dérivés d'espèces différentes, un anticorps selon la revendication 7 ou 8, une séquence d'ADN de la revendication 9 ou un vecteur d'expression selon la revendication 10 pour la préparation d'un vaccin pour la prévention ou le traitement d'une encéphalopathie spongiforme transmissible.

13. Utilisation de la revendication 12, **caractérisée en ce que** la protéine prion est une protéine prion selon l'une quelconque des revendications 1 à 5.
